# EUROPÄISCHE PATENTSCHRIFT

(11) **EP 4 191 233 B1**
(45) Veröffentlichungstag und Bekanntmachung des Hinweises auf die Patenterteilung: **01.07.2026**
(21) Anmeldenummer: 22209935.0
(22) Anmeldetag: 28.11.2022
(51) Int. Cl.: G01N 21/39, G01N 21/33, G01N 21/359, G01N 33/00, G01N 21/3504

(54) **VORRICHTUNG ZUM DETEKTIEREN EINES GASES ODER EINES MEHRKOMPONENTIGEN GASGEMISCHES**
DEVICE FOR DETECTING A GAS OR A MULTICOMPONENT GAS MIXTURE
DISPOSITIF DE DÉTECTION D'UN GAZ OU D'UN MÉLANGE GAZEUX À PLUSIEURS COMPOSANTS

(30) Priorität: 02.12.2021 DE 102021131832
(43) Veröffentlichungstag der Anmeldung: 07.06.2023
(73) Patentinhaber: Endress+Hauser Group Services AG, 4153 Reinach (CH)
(72) Erfinder: Bergau, Max, 79104 Freiburg (DE); Scherer, Benjamin, 79254 Oberried (DE)
(74) Vertreter: Endress + Hauser Group Services (Deutschland) AG+Co. KG

(56) Entgegenhaltungen:
- US-A- 5 430 293
- US-A1- 2009 159 798
- US-A1- 2020 363 327
- US-B1- 8 502 152

## Beschreibung

Die Erfindung betrifft eine Vorrichtung zum Detektieren eines Gases oder eines mehrkomponentigen Gasgemisches. Des Weiteren betrifft die Erfindung ein Verfahren zum Detektieren eines Gases oder eines mehrkomponentigen Gasgemisches mittels einer erfindungsgemäßen Vorrichtung.

Ein aufkommender Trend in der Gasdetektion ist die Detektion von Gasen aus der Entfernung.

Zu diesem Zweck gibt es sogenannte Punktsensoren und Gaskameras, wobei erstere nur die Gaskonzentration entlang einer Linie messen, während Gaskameras eine Bilddarstellung der Gaswolke liefern (vgl. die Veröffentlichungen Bonow, G.; Kroll, A.: "Zur automatisierten Inspektion von Anlagen mittels Gasfernmesstechnik: Technologien und Geräte", AUTOMATION 2011, Baden-Baden, 28.-29. Juni 2011, sowie Strahl et al., "Methane Leak detection by tunable laser spectroscopy and mid-infrared imaging," Appl. Opt. 60, C68-C71 (2021). ). Die Gaskameras werden oft als optische Gasbildgebung ("optical gas imaging", kurz OGI) bezeichnet. Zwei weitere Beispiele für Gasbildgebungssysteme werden in Patentdokumenten US 5 430 293 A und US 2009/159798 A1 genannt.

Hierfür sind heutzutage zwei Technologien etabliert, welche sich in ihrer Funktionsweise unterscheiden: die passive Detektion und die aktive Detektion.

Die passive Detektion ist das derzeitige Standardverfahren für Gaskameras zur Gasdetektion aus der Ferne. Die thermische Hintergrundstrahlung, d. h. die Infrarotstrahlung, wird von einer Infrarotkamera erfasst, die normalerweise mit einem Bandpassfilter ausgestattet ist. Der Filter wird so gewählt, dass er Infrarotlicht nur in der Nähe der Absorptionslinien des gesuchten Gases durchlässt. Wenn Gas vorhanden ist, wird das Bild an diesen Stellen "verdunkelt" oder "aufgehellt", je nach Temperatur des Gases und des Hintergrunds.

Ein weiterer Vergleich, beispielsweise mit einem Referenzbild, also mit einem Bild ganz ohne Filter oder mit einem Filter, der nicht auf den Absorptionslinien des Zielgases liegt, zeigt das detektierte Gas deutlicher. Führende Industrieunternehmen, die solche Produkte verkaufen, sind die ehemaligen "Rebellion Photonics" - jetzt "Honeywell" - oder "FLIR".

Bei der aktiven Detektion wird, anstatt die thermische Hintergrundstrahlung zu nutzen, eine eigene Lichtquelle verwendet, um den interessierenden Bereich zu beleuchten. Das eingesendete Licht wird von einem Hintergrund, der für das Funktionieren dieser Technik erforderlich ist, reflektiert. Anschließend wird das reflektierte Licht auf die gleiche Weise wie bei der passiven Erkennung ausgewertet, d. h. die Lichtintensität auf und neben der Absorptionslinie wird verglichen. Führende Industrieunternehmen sind: "Sewerin" und QLM. Diese Technologie gibt es bisher nur als Punktsensoren. Ein Beispiel für eine solche aktive Detektion ist in der Veröffentlichung K. J. Nutt, N. Hempler, G. T. Maker, G. P. A. Malcolm, M. J. Padgett, and G. M. Gibson, "Developing a portable gas imaging camera using highly tunable active-illumination and computer vision," Opt. Express 28, 18566-18576 (2020) zu finden.

Beide Technologien haben Vor- und Nachteile:
So kann der maximal mögliche Messabstand zwischen Kamera und Gas bei der passiven Erkennung mehrere 100 m betragen, während er bei der aktiven Detektion wesentlich geringer ist (ungefähr 2 m).

Bei der passiven Detektion werden an den Hintergrund keine Anforderungen gestellt, während der Hintergrund bei der aktiven Detektion im Messbereich liegen muss.

Hinsichtlich der Temperaturdifferenz werden bei der aktiven Detektion keine Anforderungen gestellt, während sich bei der passiven Detektion die Temperatur von Gas und Hintergrund unterscheiden muss.

Hinsichtlich der Gasmenge werden bei der aktiven Detektion keine Anforderungen gestellt, wobei bei der passiven Detektion beachtet werden muss, dass wenn das Gas das gesamte Sichtfeld abdeckt, es nicht erkannt werden kann.

Bei der aktiven Detektion kann das detektierte Gas kann eindeutig identifiziert werden, wobei bei der passiven Detektion typischerweise nur die Gasgruppe erkannt werden kann. Die Sensitivität beträgt bei der aktiven Detektion ungefähr 1 ppm*m für Methan), bei der passiven Detektion ungefähr 100 ppm * m für Methan.

Bei Methoden unterscheiden sich auch darin, das Vorhandensein von Gas zu detektieren. Bei der aktiven Detektion wird das Gas als solches identifiziert, während bei der passiven Detektion hierfür Algorithmik erforderlich ist. Ein Beispiel für letzteres ist in der Veröffentlichung Wang et al., 2020, "Machine vision for natural gas methane emissions detection using an infrared camera," Applied Energy, Elsevier, vol. 257(C) beschrieben, in welcher ein Deep-Learning-Algorithmus verwendet wird, um Gaswolken in einer passiven Gaskamera (OGI) zu erkennen.

Ausgehend von der beschriebenen Problematik liegt der Erfindung die Aufgabe zugrunde, die Detektionsgenauigkeit und -selektivität eines Gases, bzw. eines Gasgemischs gegenüber der passiven Detektionsmethode zu verbessern.

Die Aufgabe wird durch eine Vorrichtung zum Detektieren eines Gases oder eines mehrkomponentigen Gasgemisches gelöst, wobei die Vorrichtung umfasst:
eine optische Erfassungseinheit zum Erfassen eines Sichtfeld der optischen Erfassungseinheit, eine erste Lichtquelle ausgestaltet zum Emittieren von Licht mit einer innerhalb eines ersten Wellenlängenbereichs einstellbaren aktuellen Wellenlänge, wobei die erste Lichtquelle wahlweise ein- und ausschaltbar ist und diese derart in Relation zur optischen Erfassungseinheit angeordnet ist, dass das von der ersten Lichtquelle emittierte Licht auf das Sichtfeld der optischen Erfassungseinheit trifft, einen im Sichtfeld der optischen Erfassungseinheit und zwischen der optischen Erfassungseinheit und der ersten Lichtquelle angeordneten ersten optischen Filter, wobei der erste optische Filter insbesondere wahlweise zu- und abschaltbar ist, und wobei der erste optische Filter nur für diejenigen Wellenlängen des Lichts durchlässig ist, ersten Filterwellenlängenbereich liegen, wobei sich der erste Wellenlängenbereichs der ersten Leuchtquelle innerhalb des ersten Filterwellenlängenbereichs befindet, und eine Regel-/Auswerteeinheit, welche ausgestaltet ist, anhand des von der optischen Erfassungseinheit zumindest einen aufgenommenen Bildes das Vorhandensein des Gases, bzw. des Gasgemischs, die Verteilung des Gases, bzw. des Gasgemischs, im Sichtfeld der optischen Erfassungseinheit, die Zusammensetzung des Gases, bzw. des Gasgemischs, und/oder eine Konzentration der Komponenten des Gasgemischs zu bestimmen.

Die erfindungsgemäße Vorrichtung kombiniert die Vorteile der aktiven und der passiven Gasdetektion in einem Messgerät. Diese Kombination wird aus Sicht der Hardware durch die Erweiterung der optischen Erfassungseinheit mit einer geeigneten, abstimmbaren ersten Lichtquelle erreicht.

Bei der optischen Erfassungseinheit handelt es sich beispielsweise um eine Foto- oder Videokamera. Es kann aber auch eine Fotodiode (single pixel) oder ein Zeilendetektor verwendet werden. Diese Sensoren führen kontinuierlich einen Scan durch, indem sie den Erfassungsbereich (Fotodiode: zeilen- und spaltenweise; Zeilendetektor: zeilenweise) verändern, um das gesamte Sichtfeld erfassen zu können.

Gemäß einer vorteilhaften Ausgestaltung der erfindungsgemäßen Vorrichtung ist vorgesehen, dass die Regel-/Auswerteeinheit dazu ausgestaltet ist, ein Einstellen von zumindest einer aktuellen Wellenlänge der ersten Lichtquelle mit einer Auslösung der optischen Erfassungseinheit zur Aufnahme zumindest eines Bilds zu synchronisieren. Die optische Erfassungseinheit wird also genau dann angewiesen, ein Bild aufzunehmen, wenn die erste Lichtquelle auf die neue Wellenlänge umgestellt ist.

Gemäß einer alternativen vorteilhaften Ausgestaltung der erfindungsgemäßen Vorrichtung ist vorgesehen, dass die Regel-/Auswerteeinheit dazu ausgestaltet ist, die Lichtquelle nacheinander auf zumindest zwei voneinander verschiedene aktuelle Wellenlängen einzustellen, wobei die Lichtquelle dazu ausgestaltet ist, die optische Erfassungseinheit jeweils dann auszulösen, wenn die Lichtquelle entsprechend auf die jeweiligen aktuellen Wellenlängen eingestellt ist. Auf diese Art und Weise ist eine Absorptionslinie des zu analysierenden, bzw. zu detektierenden Gases, bzw. Gasgemisches, aufnehmbar. Anhand dieser Absorptionslinie sind weitere Eigenschaften des Gases, bzw. des Gasgemischs bestimmbar sind.

In einer Weiterbildung der erfindungsgemäßen Vorrichtung sind ein oder mehrere weitere optische Filter vorgesehen, wobei die weiteren optischen Filter jeweils einen zueinander und zum ersten Filterwellenbereich verschiedenen weiteren Filterwellenbereich aufweisen.

Gemäß einer ersten Variante ist vorgesehen, dass die Vorrichtung ein Filterrad umfasst, welches den ersten optischen Filter und den, bzw. die weiteren optischen Filter aufweist, wobei das Filterrad dazu ausgestaltet ist, durch Rotieren des Filterrads wahlweise den ersten optischen Filter, einen der weiteren optischen Filter oder keinen optischen Filter im Sichtfeld der optischen Erfassungseinheit anzuordnen.

Gemäß einer ersten Variante ist vorgesehen, dass die Vorrichtung eine Filteraufnahme zum Anordnen eines optischen Filters im Sichtfeld der optischen Erfassungseinheit aufweist, wobei wahlweise der erste optische Filter, einer der weiteren optischen Filter oder kein optischer Filter in der Filteraufnahme anordenbar ist. Die Filteraufnahme befindet sich unmittelbar vor der optischen Erfassungseinheit, oder in der optischen Erfassungseinheit, bspw. zwischen Objektiv und Fotosensor, im Falle, dass eine Kamera verwendet wird.

Eine vorteilhafte Ausgestaltung der erfindungsgemäßen Vorrichtung sieht vor, dass der erste Wellenlängenbereich der ersten Lichtquelle schmalbandig ist. Als schmalbandig wird der Wellenlängenbereich einer Lichtquelle bezeichnet, wenn die Lichtquelle eine Halbwertsbreite (FWHM) von kleiner 5 Wellenzahlen aufweist.

Gemäß einer vorteilhaften Ausgestaltung der erfindungsgemäßen Vorrichtung ist vorgesehen, dass der erste Wellenlängenbereich im Infrarotbereich oder im UV-Bereich liegt.

Gemäß einer vorteilhaften Ausgestaltung der erfindungsgemäßen Vorrichtung ist vorgesehen, dass eine oder mehrere weitere Lichtquellen vorgesehen ist, wobei die weiteren Lichtquellen jeweils einen zueinander und zum ersten Wellenlängenbereich verschiedenen weiteren Wellenlängenbereich aufweisen, wobei die weitere Lichtquelle, bzw. eine der weiteren Lichtquellen, wahlweise ein- und ausschaltbar ist und diese, bzw. die optische Erfassungseinheit, derart angeordnet ist, dass deren emittiertes Licht auf das Sichtfeld der optischen Erfassungseinheit trifft. Die erste Lichtquelle wird derart ausgerichtet, dass das emittierte Licht auf einen reflektierenden Hintergrund trifft, wobei das reflektierte Licht von der optischen Erfassungseinheit erfasst wird. Das zu analysierende Gas befindet sich dabei im Strahlengang des emittierten Lichts und/oder des reflektierten Lichts.

Gemäß einer vorteilhaften Ausgestaltung der erfindungsgemäßen Vorrichtung ist vorgesehen, dass die erste Lichtquelle, bzw. die weiteren Lichtquellen, ein durchstimmbarer Laser, insbesondere ein Diodenlaser oder ein Quantenkaskadenlaser, ist.

Gemäß einer vorteilhaften Ausgestaltung der erfindungsgemäßen Vorrichtung ist vorgesehen, dass eine optische Manipulationseinheit vorgesehen ist, welche derart ausgestaltet ist, dass diese das von der ersten Lichtquelle, bzw. von der weiteren Lichtquelle, bzw. von einer der weiteren Lichtquellen emittierte Licht derart aufweitet, dass es auf das komplette Sichtfeld der optischen Erfassungseinheit trifft, oder welche derart ausgestaltet ist, dass das von der ersten Lichtquelle emittierte Licht punktförmig vorliegt und rasterförmig über das Sichtfeld der optischen Erfassungseinheit gefahren wird, wobei die Regel-/Auswerteeinheit ausgestaltet ist, eine Vielzahl von Bildern an verschiedenen Positionen des Lichts im Raster aufzunehmen. Mittels beider Methoden ist eine komplette Erfassung des Sichtfelds der optischen Erfassungseinheit realisierbar.

Des Weiteren wird die Aufgabe durch ein Verfahren zum Detektieren eines Gases oder eines mehrkomponentigen Gasgemisches mittels einer erfindungsgemäßen Vorrichtung gelöst, umfassend:
- Betreiben der Vorrichtung in einem passiven Detektionsmodus, wobei die erste Lichtquelle im passiven Detektionsmodus ausgeschaltet ist und die optische Erfassungseinheit ein erstes Bild erfasst,
- Auswerten des erfassten ersten Bildes und Bestimmen der Position eines Gases, bzw. eines Gasgemischs im Sichtfeld der optischen Erfassungseinheit,
- Betreiben der Vorrichtung in einem aktiven Detektionsmodus, wobei die erste Lichtquelle im aktiven Detektionsmodus eingeschaltet ist und Licht mit einer ersten aktuellen Wellenlänge emittiert:
   i. Ausrichten der Vorrichtung mit der optischen Erfassungseinheit auf die bestimmte Position und Erfassen eines zweiten Bilds mittels der optischen Erfassungseinheit;
   ii. Erfassen eines dritten Bilds mittels der optischen Erfassungseinheit an derselben Position, wobei die erste Lichtquelle eine zweite aktuelle Wellenlänge, welche zur ersten aktuellen Wellenlänge verschieden ist, emittiert;
- Bestimmen der Zusammensetzung des Gases, bzw. des Gasgemischs, bzw. Identifizieren des Gases, bzw. des Gasgemischs durch Auswerten des zweiten Bildes und des dritten Bildes.

Erfindungsgemäß wird somit zuerst die Position des Gases, bzw. des Gasgemischs bestimmt und anschließend, in dem aktiven Detektionsmodus, Eigenschaften des detektierten Gases, bzw. Gasgemischs bestimmt. Es ist hierbei vorgesehen, dass die erste aktuelle Wellenlänge ausreichend von derjenigen Wellenlänge, welche von dem Gas, bzw. dem Gasgemisch maximal absorbiert wird, derart weit entfernt ist, dass unter Normalbedingungen keine Absorption oder eine verminderte Absorption (bspw. 10 % der maximalen Absorption) des von der ersten Lichtquelle emittierten Lichts auftritt. Das derart erfasste zweite Bild wird als Referenzbild verwendet. Die zweite aktuelle Wellenlänge wird derart gewählt, dass unter Normalbedingungen eine maximale Absorption des von der ersten Lichtquelle emittierten Lichts durch das Gas, bzw. das Gasgemisch auftritt. Das erfasste dritte Bild wird durch Bildung eines Differenzbilds mit dem Referenzbild verglichen und die Eigenschaften des Gases, bzw. des Gasgemischs durch die Auswertung beider Bilder, bzw. deren Vergleich, bestimmt.

Gemäß einer vorteilhaften Ausgestaltung des erfindungsgemäßen Verfahrens ist vorgesehen, dass die Lichtquelle nacheinander die erste aktuelle Wellenlänge und ein oder mehrere weitere aktuelle Wellenlängen emittiert, wobei die optische Erfassungseinheit mindestens ein weiteres Bild bei Vorliegen jeder der weiteren aktuellen Wellenlängen aufnimmt, wobei das weitere Bild, bzw. die weiteren Bilder für das Bestimmen der Zusammensetzung des Gases, bzw. des Gasgemischs, bzw. Identifizieren des Gases, bzw. des Gasgemischs mit ausgewertet werden. Es wird somit ein sogenannter "Sweep" der ersten Lichtquelle durchgeführt. Dadurch wird eine Spektrallinie des Gases, bzw. des Gasgemischs aufgezeichnet, anhand derer die Eigenschaften des Gases, bzw. des Gasgemischs, bestimmbar sind.

Gemäß einer vorteilhaften Ausgestaltung des erfindungsgemäßen Verfahrens ist vorgesehen, dass anschließend sukzessive die eine oder mehrere der weiteren Lichtquellen eingeschaltet wird, wobei insbesondere nacheinander mehrere aktuelle Wellenlängen eingestellt werden, und wobei die optische Erfassungseinheit ein oder mehrere weitere aktuelle Bilder aufnimmt, wobei das weitere Bild, bzw. die weiteren Bilder für das Bestimmen der Zusammensetzung des Gases, bzw. des Gasgemischs, bzw. Identifizieren des Gases, bzw. des Gasgemischs mit ausgewertet werden. Gemäß einer vorteilhaften Ausgestaltung des erfindungsgemäßen Verfahrens ist vorgesehen, dass für jede der weiteren Lichtquellen einer der weiteren optischen Filter zugeschaltet wird. Es ist hierbei vorgesehen, dass die jeweiligen Wellenlängenbereiche der weiteren Lichtquellen in den Filterwellenlängenbereichen des jeweils entsprechenden weiteren optischen Filter liegen.

Die Erfindung wird anhand der nachfolgenden Figur näher erläutert. Es zeigt
Fig. 1: eine schematische Darstellung eines ersten Ausführungsbeispiels der erfindungsgemäßen Vorrichtung; und
Fig. 2: eine schematische Darstellung eines zweiten Ausführungsbeispiels der erfindungsgemäßen Vorrichtung.

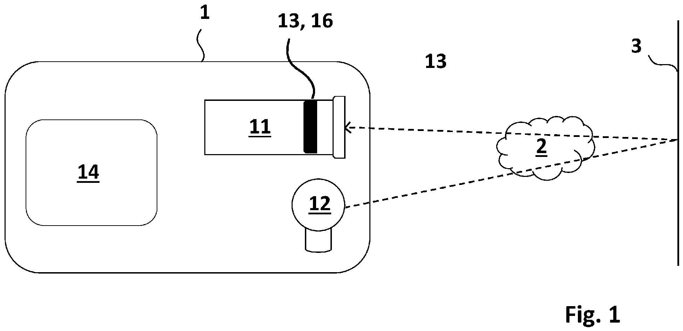

In Fig. 1 ist ein erstes Ausführungsbeispiel der erfindungsgemäßen Vorrichtung 1, bzw. des erfindungsgemäßen Verfahrens gezeigt.

Die Vorrichtung 1 besteht im Wesentlichen aus einer optischen Erfassungseinheit 11, im vorliegenden Fall eine passive Gaskamera, das heißt, eine Infrarotkamera mit einem oder mehreren optischen Filtern 13, 13', welche insbesondere gekühlt sind. Die optische Erfassungseinheit 11 wird durch eine erste Lichtquelle 12 erweitert. Bei der ersten Lichtquelle 12 handelt es sich um eine monochromatische Lichtquelle, insbesondere um einen Laser oder eine ähnliche geeignete Lichtquelle. Die erste Lichtquelle 12 ist dazu ausgestaltet, Licht mit einer aktuellen Wellenlänge zu emittieren, welche aktuelle Wellenlänge innerhalb eines ersten, insbesondere schmalbandigen, Wellenlängenbereichs liegt. Die erste Lichtquelle ist insbesondere durchstimmbar, das heißt, die aktuelle Wellenlänge ist innerhalb des ersten Wellenlängenbereichs verschiebbar. Des Weiteren ist eine Regel-/Auswerteeinheit 14 vorgesehen, welche die optische Erfassungseinheit 11 steuern, also Video- und Bildaufnahmen initiieren kann, sowie die erste Lichtquelle 12 ein- und ausschalten, sowie die aktuelle Wellenlänge des von der ersten Lichtquelle 12 emittierten Lichts einstellen kann.

Die Vorrichtung wird nacheinander zuerst in einem passiven Detektionsmodus und anschließend in einem aktiven Detektionsmodus betrieben.

Im passiven Detektionsmodus ist die erste Lichtquelle 12 ausgeschaltet. Die Regel-/Auswerteeinheit 14 weist die optische Erfassungseinheit 11 an, ein erstes Bild von deren Sichtfeld aufzunehmen. In der optischen Erfassungseinheit 11 ist ein erster optischer Filter angebracht, insbesondere in einer entsprechenden Filteraufnahme 16 der optischen Erfassungseinheit 11, welcher optische Filter 13 im Strahlengang des einfallendes Lichts angeordnet wird. Der erste optische Filter ist ein Bandpassfilter, welcher Infrarotlicht nur in der Nähe der Absorptionslinien des gesuchten Gases 2 durchlässt. Im erfassten ersten Bild wird somit eventuell vorhandenes Gas 2 optische von dem Hintergrund getrennt.

Das erste Bild wird von der Regel-/Auswerteeinheit 14 oder einer externen Einheit, bspw. einem PC, mittels Algorithmik, bspw. Bilderkennungsalgorithmen, analysiert und das Vorhandensein und insbesondere die Position des Gases 2 bestimmt.

Da Gas häufig als Gemisch vorliegt, dessen Zusammensetzung, bzw. Konzentration vielfältig sein kann, wird die Vorrichtung 1 anschließend in einem aktiven Detektionsmodus betrieben. Hierfür wird die Vorrichtung auf das Gas, bzw. das Gasgemisch 2 ausgerichtet. Die Position des Gases 2 ist aus dem analysierten ersten Bild erhalten worden. Im Strahlengang des emittierten Lichts und/oder des reflektierten Lichts, befindet sich nun das zu analysierende Gas, bzw. Gasgemisch. Für den aktiven Detektionsmodus wird die erste Lichtquelle 12 eingeschaltet und auf eine erste aktuelle Wellenlänge eingestellt, die außerhalb der Gaslinie des Gases, bzw. des Gasgemischs 2 liegt, d.h. ausreichend von derjenigen Wellenlänge entfernt liegt, bei welcher das von der ersten Lichtquelle 12 emittierte Licht maximal von dem Gas, bzw. dem Gasgemisch 2 absorbiert wird. Das von der ersten Leuchtquelle 12 emittierte Licht verlässt die Vorrichtung 1, wird derart an einem Hintergrund reflektiert, so dass reflektierte Licht von der optischen Erfassungseinheit erfasst wird. Die Regel-/Auswerteeinheit 14 weist die optische Erfassungseinheit 11 an, ein zweites Bild von deren Sichtfeld aufzunehmen.

Die Regel-/Auswerteeinheit 14 weist die optische Erfassungseinheit 11 an, ein drittes Bild von deren Sichtfeld aufzunehmen. Hierfür wird die erste Lichtquelle 12 auf eine zweite aktuelle Wellenlänge eingestellt, bei welcher das von der ersten Lichtquelle 12 emittierte Licht maximal von dem Gas, bzw. dem Gasgemisch 2 absorbiert wird

Durch Analysieren des zweiten und des dritten Bildes, insbesondere durch Bilden eines Differenzbilds, kann die Regel-/Auswerteeinheit 14, bzw. eine externe Einheit, die Zusammensetzung des Gases, bzw. des Gasgemischs bestimmen und/oder das Gas, bzw. das Gasgemisch 2 identifizieren. Dies ist dadurch möglich, dass die aktuelle Wellenlänge des von der ersten Lichtquelle emittierten Lichts derart eingestellt wird, dass sich diese im Absorptionsspektrum des Gases 2 befindet.

Zur genaueren Bestimmung können von der optischen Erfassungseinheit 11 weitere Bilder aufgenommen werden. Die erste Lichtquelle wird derart angesteuert, dass die aktuelle Wellenlänge für jedes weitere aufzunehmende Bild verschoben wird. Dadurch wird eine Absorptionslinie des Gases erhoben, anhand welcher die Eigenschaften des Gases, bzw. des Gasgemischs 2 genau bestimmbar sind. Für jedes Gasgemisch wird die Absorptionslinie verschieden aussehen. Die Absorptionslinie zeigt lokale Minima an denjenigen Wellenlängen auf, welche der Absorptionswellenlänge der entsprechenden Gaskomponente entspricht.

Gemäß der Erfindung werden zu einem Zeitpunkt, bei welchem die optische Erfassungseinheit 11 neben das Gas 2 gerichtet ist, ebenfalls entsprechende Bilder für verschiedene aktuelle Wellenlängen des von der ersten Lichtquelle 12 emittierten Lichts auszusenden. Dieses Referenzspektrum wird für die Analyse des Gases, bzw. des Gasgemischs 2 verwendet.

Um plausible Resultate erhalten zu können, müssen bestimmte Anforderungen von dem optischen Filter 13 und der ersten Lichtquelle 11 erfüllt werden:
Der spektrale Erfassungsbereich muss die gewählten Absorptionslinien des Zielgases abdecken.

Weiterhin muss die aktuelle Wellenlänge, bzw. der erste Wellenlängenbereich, des von der ersten Lichtquelle emittierten Lichts von der optischen Erfassungseinheit 11 erkannt werden. Der erste optische Filter muss also so gewählt werden, dass dieser für Licht im ersten Wellenlängenbereich durchlässig ist. Im Falle, dass der optische Filter einen Filterwellenlängenbereich im mittleren Infrarotbereich aufweist, muss der erste optische Filter gekühlt werden, um möglichst wenig thermische Abstrahlung des ersten optischen Filters auf den Detektor der optischen Erfassungseinheit 11 zu haben. Befindet sich der Filterwellenlängenbereich im Nah-Infrarotbereich oder im ultravioletten Bereich, so wird eine Kühlung nicht benötigt. Die aktuelle Wellenlänge, bzw. der erste Wellenlängenbereich muss außerdem so gewählt werden, dass dieser mit einer eindeutigen Absorptionslinie des Zielgases übereinstimmt. Hierbei sollte keine spektrale Überlappung mit einem anderen Gas erfolgen, um eine gasspezifische Detektion zu ermöglichen.

Die erfindungsgemäße Vorrichtung kombiniert die Vorteile der aktiven und der passiven Gasdetektion. In einem Anwendungsbeispiel ist die Vorrichtung 1 Teil eines Roboters, welcher in einer industriellen Anlage eingesetzt ist. Die optische Erfassungseinheit 11 detektiert im passiven Detektionsmodus eine Gaswolke aus 50 Metern Entfernung. Die Regel-/Auswerteeinheit 14 identifiziert deren Position. Die genauen Eigenschaften des Gases können aber anhand des passiven Detektionsmodus alleine noch nicht quantifiziert werden. Der Roboter nähert sich der detektierten Position, wobei die Vorrichtung in den aktiven Detektionsmodus wechselt. In diesem Modus bestätigt die Regel-/Auswerteeinheit 14, dass es sich um austretendes Methan handelt. Es wird hierfür eine Säulenkonzentration von 1000 ppm*m aus 1 Meter Abstand zur Rohrleitung gemessen. Falls die optische Weglänge nicht weiter eingegrenzt werden kann, wird eine Mindestkonzentration von 500 ppm übermittelt (1000 ppm*m / (2 * 1 m)). Im vorliegenden Beispiel wird ein Absorptionsmaximum bei 3,267 µm ermittelt, woran die Vorrichtung durch Vergleich mit bekannten Werten das Gas Methan identifiziert. Methan weist nämlich beispielsweise große Absorptionslinien im Bereich von ca. 1,6 µm (Nahinfrarot), 2,3 µm und 3,3 µm (Mid-Infrarot) auf. Mittels intelligenter Algorithmik kann die Regel-/Auswerteeinheit 14 anhand der aufgenommenen Bilder austretende Gasmenge quantifizieren und ggf. auch die Leckquelle (bspw. in einer Rohrleitung oder an einer Wand eines Behälters) aufspüren. Der aktive Ansatz funktioniert auch dann, wenn die Gaswolke das gesamte Bild verdeckt, während dies mittels der passiven Detektion nicht möglich wäre.

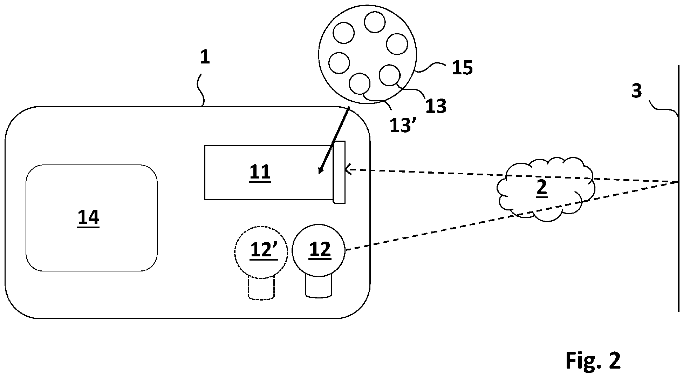

Fig. 2 zeigt ein zweites Ausführungsbeispiel der erfindungsgemäßen Vorrichtung. Zur Detektion verschiedener Gastypen weist die Vorrichtung eine oder mehrere weitere Lichtquellen 13' auf, die beliebig von der Regel-/Auswerteeinheit ein- und ausgeschaltet werden können. Jeder der weiteren Lichtquellen 13' ist ein weiterer optischer Filter 13' zugeordnet. Vorteilhafterweise sind die optischen Filter 13, 13' in einem, vorzugsweise gekühlten, Filterrad angeordnet, welches durch Rotieren auf komfortable Weise einen der Filter 13, 13' im Strahlengang des in der optischen Erfassungseinheit 11 einfallenden Lichts anordnet.

Es können nun sukzessive die eine oder mehrere der weiteren Lichtquellen eingeschaltet werden, wobei insbesondere nacheinander mehrere aktuelle Wellenlängen eingestellt werden, und wobei die optische Erfassungseinheit ein oder mehrere weitere aktuelle Bilder aufnimmt, wobei das weitere Bild, bzw. die weiteren Bilder für das Bestimmen der Zusammensetzung des Gases, bzw. des Gasgemischs (2), bzw. Identifizieren des Gases, bzw. des Gasgemischs (2) mit ausgewertet werden. Hierdurch können verschiedenste Gasgemische mit Gasanteilen verschiedenster spektraler Eigenschaften analysiert und bestimmt werden.

### Bezugszeichenliste

- 1: Vorrichtung
- 11: optische Erfassungseinheit
- 12: erste Lichtquelle
- 12': weitere Lichtquelle
- 13: erster optischer Filter
- 13': weitere optische Filter
- 14: Regel-/Auswerteeinheit
- 15: Filterrad
- 16: Filteraufnahme
- 2: Gas, Gasgemisch
- 3: reflektierender Hintergrund

## Patentansprüche

1. Vorrichtung (1) zum Detektieren eines Gases oder eines mehrkomponentigen Gasgemisches (2) , umfassend eine optische Erfassungseinheit (11) zum Erfassen eines Sichtfeld der optischen Erfassungseinheit (11), eine erste Lichtquelle (12) ausgestaltet zum Emittieren von Licht mit einer innerhalb eines ersten Wellenlängenbereichs einstellbaren aktuellen Wellenlänge, wobei die erste Lichtquelle (12) wahlweise ein- und ausschaltbar ist und diese derart in Relation zur optischen Erfassungseinheit (11) angeordnet ist, dass das von der ersten Lichtquelle (12) emittierte Licht auf das Sichtfeld der optischen Erfassungseinheit (11) trifft, einen im Sichtfeld der optischen Erfassungseinheit (11) und zwischen der optischen Erfassungseinheit (11) und der ersten Lichtquelle (12) angeordneten ersten optischen Filter (13), wobei der erste optische Filter (13) insbesondere wahlweise zu- und abschaltbar ist, und wobei der erste optische Filter (13) nur für diejenigen Wellenlängen des Lichts durchlässig ist, welche im ersten Filterwellenlängenbereich liegen, wobei sich der erste Wellenlängenbereich der ersten Leuchtquelle innerhalb des ersten Filterwellenlängenbereichs befindet, und eine Regel-/Auswerteeinheit (14), welche ausgestaltet ist, anhand des von der optischen Erfassungseinheit (11) zumindest einen aufgenommenen Bildes das Vorhandensein des Gases, bzw. des Gasgemischs (2), die Verteilung des Gases, bzw. des Gasgemischs (2), im Sichtfeld der optischen Erfassungseinheit (11), die Zusammensetzung des Gases, bzw. des Gasgemischs (2), und/oder eine Konzentration der Komponenten des Gasgemischs (2) zu bestimmen, indem die Regel-/Auswerteeinheit die folgenden Schritte veranlasst:
a. Betreiben der Vorrichtung (1) in einem passiven Detektionsmodus, wobei die erste Lichtquelle(12) im passiven Detektionsmodus ausgeschaltet ist und die optische Erfassungseinheit (11) ein erstes Bild erfasst,
b. Auswerten des erfassten ersten Bildes und Bestimmen der Position eines Gases, bzw. eines Gasgemischs (2) im Sichtfeld der optischen Erfassungseinheit (11),
c. Betreiben der Vorrichtung (1) in einem aktiven Detektionsmodus, wobei die erste Lichtquelle (12) im aktiven Detektionsmodus eingeschaltet ist und Licht mit einer ersten aktuellen Wellenlänge emittiert:
i. Ausrichten der Vorrichtung (1) mit der optischen Erfassungseinheit (11) auf die bestimmte Position und Erfassen eines zweiten Bilds mittels der optischen Erfassungseinheit (11);
ii. Erfassen eines dritten Bilds mittels der optischen Erfassungseinheit (11) an der bestimmten Position, wobei die erste Lichtquelle (12) eine zweite aktuelle Wellenlänge, welche zur ersten aktuellen Wellenlänge verschieden ist, emittiert;
d. Bestimmen der Zusammensetzung des Gases, bzw. des Gasgemischs (2), bzw. Identifizieren des Gases, bzw. des Gasgemischs (2) durch Auswerten des zweiten Bildes und des dritten Bildes, wobei die Lichtquelle (12) nacheinander die erste aktuelle Wellenlänge und ein oder mehrere weitere aktuelle Wellenlängen emittiert, wobei die optische Erfassungseinheit (11) eine Absorptionslinie des Gases, bzw. des Gasgemischs erhebt, anhand welcher die Eigenschaften des Gases, bzw. des Gasgemischs (2) genau bestimmbar sind, indem mindestens ein weiteres Bild bei Vorliegen jeder der weiteren aktuellen Wellenlängen aufgenommen wird, wobei das weitere Bild, bzw. die weiteren Bilder für das Bestimmen der Zusammensetzung des Gases, bzw. des Gasgemischs (2), bzw. Identifizieren des Gases, bzw. des Gasgemischs (2) mit ausgewertet werden, wobei zum Erfassen eines Referenzspektrums die optische Erfassungseinheit neben das Gas, bzw. das Gasgemisch (2) gerichtet wird und ebenfalls entsprechende Bilder für verschiedene aktuelle Wellenlängen erfasst werden, wobei das Referenzspektrum für das Bestimmen der Zusammensetzung des Gases, bzw. des Gasgemischs (2), bzw. Identifizieren des Gases, bzw. des Gasgemischs (2) der Gases, bzw. des Gasgemischs (2) verwendet wird.

2. Vorrichtung (1) nach Anspruch 1, wobei die Regel-/Auswerteeinheit (14) dazu ausgestaltet ist, ein Einstellen von zumindest einer aktuellen Wellenlänge der ersten Lichtquelle (12) mit einer Auslösung der optischen Erfassungseinheit (11) zur Aufnahme zumindest eines Bilds zu synchronisieren.

3. Vorrichtung (1) nach Anspruch 1, wobei die Regel-/Auswerteeinheit (14) dazu ausgestaltet ist, die erste Lichtquelle (12) nacheinander auf zumindest zwei voneinander verschiedene aktuelle Wellenlängen einzustellen, wobei die erste Lichtquelle dazu ausgestaltet ist, die optische Erfassungseinheit (11) jeweils dann auszulösen, wenn die erste Lichtquelle (12) entsprechend auf die jeweiligen aktuellen Wellenlängen eingestellt ist.

4. Vorrichtung (1) nach zumindest einem der vorherigen Ansprüche, wobei die Vorrichtung (1) einen oder mehrere weitere optische Filter (13') aufweist, wobei die weiteren optischen Filter (13') jeweils einen zueinander und zum ersten Filterwellenbereich verschiedenen weiteren Filterwellenbereich aufweisen.

5. Vorrichtung (1) nach Anspruch 4, weiter umfassend ein Filterrad (15), welches den ersten optischen Filter (13) und den, bzw. die weiteren optischen Filter (13, 13') aufweist, wobei das Filterrad (15) dazu ausgestaltet ist, durch Rotieren des Filterrads (15) wahlweise den ersten optischen Filter (13), einen der weiteren optischen Filter (13') oder keinen optischen Filter im Sichtfeld der optischen Erfassungseinheit (11) anzuordnen.

6. Vorrichtung (1) nach Anspruch 4, wobei die Vorrichtung (1) eine Filteraufnahme (16) zum Anordnen eines optischen Filters (13, 13') im Sichtfeld der optischen Erfassungseinheit (11) aufweist, wobei wahlweise der erste optische Filter (13), einer der weiteren optischen Filter (13') oder kein optischer Filter in der Filteraufnahme (16) anordenbar ist.

7. Vorrichtung (1) nach zumindest einem der vorherigen Ansprüche, wobei der erste Wellenlängenbereich der ersten Lichtquelle (12) schmalbandig ist.

8. Vorrichtung (1) nach zumindest einem der vorherigen Ansprüche, wobei der erste Wellenlängenbereich im Infrarotbereich oder im UV-Bereich liegt.

9. Vorrichtung (1) nach zumindest einem der vorherigen Ansprüche, weiter umfassend eine oder mehrere weitere Lichtquellen (12'), wobei die weiteren Lichtquellen (12') jeweils einen zueinander und zum ersten Wellenlängenbereich verschiedenen weiteren Wellenlängenbereich aufweisen, wobei die weitere Lichtquelle (12'), bzw. eine der weiteren Lichtquellen (12'), wahlweise ein- und ausschaltbar ist und diese, bzw. die optische Erfassungseinheit (11), derart angeordnet ist, dass deren emittiertes Licht auf das Sichtfeld der optischen Erfassungseinheit (11) trifft.

10. Vorrichtung (1) nach zumindest einem der vorherigen Ansprüche, wobei die erste Lichtquelle (12), bzw. die weiteren Lichtquellen (12'), ein durchstimmbarer Laser, insbesondere ein Diodenlaser oder ein Quantenkaskadenlaser, ist.

11. Vorrichtung (1) nach zumindest einem der vorherigen Ansprüche, umfassend eine optische Manipulationseinheit, welche derart ausgestaltet ist, dass diese das von der ersten Lichtquelle (12), bzw. von der weiteren Lichtquelle (12'), bzw. von einer der weiteren Lichtquellen (12' )emittierte Licht derart aufweitet, dass es auf das komplette Sichtfeld der optischen Erfassungseinheit (11) trifft, oder welche derart ausgestaltet ist, dass das von der ersten Lichtquelle (12) emittierte Licht punktförmig vorliegt und rasterförmig über das Sichtfeld der optischen Erfassungseinheit (11) gefahren wird, wobei die Regel-/Auswerteeinheit (14) ausgestaltet ist, eine Vielzahl von Bildern an verschiedenen Positionen des Lichts im Raster aufzunehmen.

12. Verfahren zum Detektieren eines Gases oder eines mehrkomponentigen Gasgemisches (2) mittels einer Vorrichtung (1) nach einem der Ansprüche 1 bis 11, umfassend:
- Betreiben der Vorrichtung (1) in einem passiven Detektionsmodus, wobei die erste Lichtquelle(12) im passiven Detektionsmodus ausgeschaltet ist und die optische Erfassungseinheit (11) ein erstes Bild erfasst,
- Auswerten des erfassten ersten Bildes und Bestimmen der Position eines Gases, bzw. eines Gasgemischs (2) im Sichtfeld der optischen Erfassungseinheit (11),
- Betreiben der Vorrichtung (1) in einem aktiven Detektionsmodus, wobei die erste Lichtquelle (12) im aktiven Detektionsmodus eingeschaltet ist und Licht mit einer ersten aktuellen Wellenlänge emittiert:
i. Ausrichten der Vorrichtung (1) mit der optischen Erfassungseinheit (11) auf die bestimmte Position und Erfassen eines zweiten Bilds mittels der optischen Erfassungseinheit (11);
ii. Erfassen eines dritten Bilds mittels der optischen Erfassungseinheit (11) an der bestimmten Position, wobei die erste Lichtquelle (12) eine zweite aktuelle Wellenlänge, welche zur ersten aktuellen Wellenlänge verschieden ist, emittiert;
- Bestimmen der Zusammensetzung des Gases, bzw. des Gasgemischs (2), bzw. Identifizieren des Gases, bzw. des Gasgemischs (2) durch Auswerten des zweiten Bildes und des dritten Bildes, wobei die Lichtquelle (12) nacheinander die erste aktuelle Wellenlänge und ein oder mehrere weitere aktuelle Wellenlängen emittiert, wobei die optische Erfassungseinheit (11) eine Absorptionslinie des Gases, bzw. des Gasgemischs erhebt, anhand welcher die Eigenschaften des Gases, bzw. des Gasgemischs (2) genau bestimmbar sind, indem mindestens ein weiteres Bild bei Vorliegen jeder der weiteren aktuellen Wellenlängen aufgenommen wird, wobei das weitere Bild, bzw. die weiteren Bilder für das Bestimmen der Zusammensetzung des Gases, bzw. des Gasgemischs (2), bzw. Identifizieren des Gases, bzw. des Gasgemischs (2) mit ausgewertet werden. wobei zum Erfassen eines Referenzspektrums die optische Erfassungseinheit neben das Gas, bzw. das Gasgemisch (2) gerichtet wird und ebenfalls entsprechende Bilder für verschiedene aktuelle Wellenlängen erfasst werden, wobei das Referenzspektrum für das Bestimmen der Zusammensetzung des Gases, bzw. des Gasgemischs (2), bzw. Identifizieren des Gases, bzw. des Gasgemischs (2) der Gases, bzw. des Gasgemischs (2) verwendet wird.

13. Verfahren nach Anspruch 12, wobei anschließend sukzessive die eine oder mehrere der weiteren Lichtquellen (12') eingeschaltet wird, wobei insbesondere nacheinander mehrere aktuelle Wellenlängen eingestellt werden, und wobei die optische Erfassungseinheit (11) ein oder mehrere weitere aktuelle Bilder aufnimmt, wobei das weitere Bild, bzw. die weiteren Bilder für das Bestimmen der Zusammensetzung des Gases, bzw. des Gasgemischs (2), bzw. Identifizieren des Gases, bzw. des Gasgemischs (2) mit ausgewertet werden.

14. Verfahren nach zumindest einem der Ansprüche 12 oder 13, wobei für jede der weiteren Lichtquellen (12') einer der weiteren optischen Filter zugeschaltet wird.

## Claims

1. Device (1) for detecting a gas or a multicomponent gas mixture (2), comprising an optical detection unit (11) for capturing a field of view of the optical detection unit (11), a first light source (12) designed to emit light having a current wavelength adjustable within a first wavelength range, wherein the first light source (12) can be selectively switched on and off and is arranged relative to the optical detection unit (11) such that the light emitted by the first light source (12) impinges on the field of view of the optical detection unit (11), a first optical filter (13) arranged in the field of view of the optical detection unit (11) and between the optical detection unit (11) and the first light source (12), wherein the first optical filter (13) can in particular be selectively switched on and off, and wherein the first optical filter (13) is transmissive only for those wavelengths of the light that lie within the first filter wavelength range, wherein the first wavelength range of the first light source is within the first filter wavelength range, and a control/evaluation unit (14) which is designed to determine, on the basis of at least one image captured by the optical detection unit (11), the presence of the gas or gas mixture (2), the distribution of the gas or gas mixture (2) in the field of view of the optical detection unit (11), the composition of the gas or gas mixture (2), and/or a concentration of the components of the gas mixture (2), by causing the control/evaluation unit to perform the following steps:
a. operating the device (1) in a passive detection mode, wherein the first light source (12) is switched off in the passive detection mode and the optical detection unit (11) captures a first image,
b. evaluating the captured first image and determining the position of a gas or gas mixture (2) in the field of view of the optical detection unit (11),
c. operating the device (1) in an active detection mode, wherein the first light source (12) is switched on in the active detection mode and emits light having a first current wavelength:
i. aligning the device (1) with the optical detection unit (11) to the determined position and capturing a second image by means of the optical detection unit (11);
ii. capturing a third image by means of the optical detection unit (11) at the determined position, wherein the first light source (12) emits a second current wavelength different from the first current wavelength;
d. determining the composition of the gas or gas mixture (2), or identifying the gas or gas mixture (2), by evaluating the second image and the third image, wherein the light source (12) successively emits the first current wavelength and one or more further current wavelengths, wherein the optical detection unit (11) records an absorption line of the gas or gas mixture on the basis of which the properties of the gas or gas mixture (2) can be determined precisely, in that at least one further image is captured when each of the further current wavelengths is present, wherein the further image or the further images are also evaluated for determining the composition of the gas or gas mixture (2) or identifying the gas or gas mixture (2).

2. Device (1) according to claim 1, wherein the control/evaluation unit (14) is designed to synchronize an adjustment of at least one current wavelength of the first light source (12) with a triggering of the optical detection unit (11) for capturing at least one image.

3. Device (1) according to claim 1, wherein the control/evaluation unit (14) is designed to set the first light source (12) successively to at least two different current wavelengths, wherein the first light source is designed to trigger the optical detection unit (11) each time when the first light source (12) is correspondingly set to the respective current wavelengths.

4. Device (1) according to at least one of the preceding claims, wherein the device (1) has one or more further optical filters (13'), wherein the further optical filters (13') each have a further filter wavelength range different from one another and from the first filter wavelength range.

5. Device (1) according to claim 4, further comprising a filter wheel (15), which has the first optical filter (13) and the further optical filter or filters (13, 13'), wherein the filter wheel (15) is designed, by rotating the filter wheel (15), to selectively arrange the first optical filter (13), one of the further optical filters (13'), or no optical filter in the field of view of the optical detection unit (11).

6. Device (1) according to claim 4, wherein the device (1) has a filter holder (16) for arranging an optical filter (13, 13') in the field of view of the optical detection unit (11), wherein the first optical filter (13), one of the further optical filters (13'), or no optical filter can optionally be arranged in the filter holder (16).

7. Device (1) according to at least one of the preceding claims, wherein the first wavelength range of the first light source (12) is narrow-band.

8. Device (1) according to at least one of the preceding claims, wherein the first wavelength range lies in the infrared range or in the UV range.

9. Device (1) according to at least one of the preceding claims, further comprising one or more further light sources (12'), wherein the further light sources (12') each have a further wavelength range different from one another and from the first wavelength range, wherein the further light source (12'), or one of the further light sources (12'), can be selectively switched on and off and is arranged, or the optical detection unit (11) is arranged, such that the light emitted thereby impinges on the field of view of the optical detection unit (11).

10. Device (1) according to at least one of the preceding claims, wherein the first light source (12), or the further light sources (12'), is a tunable laser, in particular a diode laser or a quantum cascade laser.

11. Device (1) according to at least one of the preceding claims, comprising an optical manipulation unit designed such that it expands the light emitted by the first light source (12), by the further light source (12'), or by one of the further light sources (12') such that it impinges on the complete field of view of the optical detection unit (11), or designed such that the light emitted by the first light source (12) is present in point form and is guided in a raster pattern over the field of view of the optical detection unit (11), wherein the control/evaluation unit (14) is designed to capture a plurality of images at different positions of the light in the raster.

12. Method for detecting a gas or a multicomponent gas mixture (2) by means of a device (1) according to one of claims 1 to 11, comprising:
- operating the device (1) in a passive detection mode, wherein the first light source (12) is switched off in the passive detection mode and the optical detection unit (11) captures a first image,
- evaluating the captured first image and determining the position of a gas or gas mixture (2) in the field of view of the optical detection unit (11),
- operating the device (1) in an active detection mode, wherein the first light source (12) is switched on in the active detection mode and emits light having a first current wavelength:
i. aligning the device (1) with the optical detection unit (11) to the determined position and capturing a second image by means of the optical detection unit (11);
ii. capturing a third image by means of the optical detection unit (11) at the determined position, wherein the first light source (12) emits a second current wavelength different from the first current wavelength;
- determining the composition of the gas or gas mixture (2), or identifying the gas or gas mixture (2), by evaluating the second image and the third image, wherein the light source (12) successively emits the first current wavelength and one or more further current wavelengths, wherein the optical detection unit (11) records an absorption line of the gas or gas mixture on the basis of which the properties of the gas or gas mixture (2) can be determined precisely, in that at least one further image is captured when each of the further current wavelengths is present, wherein the further image or the further images are also evaluated for determining the composition of the gas or gas mixture (2) or identifying the gas or gas mixture (2).

13. Method according to claim 12, wherein subsequently the one or more further light sources (12') are switched on successively, wherein in particular a plurality of current wavelengths are set one after another, and wherein the optical detection unit (11) captures one or more further current images, wherein the further image or the further images are also evaluated for determining the composition of the gas or gas mixture (2) or identifying the gas or gas mixture (2).

14. Method according to at least one of claims 12 or 13, wherein, for each of the further light sources (12'), one of the further optical filters is switched on.

## Revendications

1. Dispositif (1) de détection d'un gaz ou d'un mélange gazeux multicomposant (2), comprenant une unité de détection optique (11) destinée à saisir un champ de vision de l'unité de détection optique (11), une première source lumineuse (12) configurée pour émettre de la lumière ayant une longueur d'onde actuelle réglable dans une première plage de longueurs d'onde, la première source lumineuse (12) pouvant être mise en marche et arrêtée sélectivement et étant disposée par rapport à l'unité de détection optique (11) de telle sorte que la lumière émise par la première source lumineuse (12) atteigne le champ de vision de l'unité de détection optique (11), un premier filtre optique (13) disposé dans le champ de vision de l'unité de détection optique (11) et entre l'unité de détection optique (11) et la première source lumineuse (12), le premier filtre optique (13) pouvant notamment être activé et désactivé sélectivement, et le premier filtre optique (13) n'étant perméable qu'aux longueurs d'onde de la lumière situées dans une première plage de longueurs d'onde du filtre, la première plage de longueurs d'onde de la première source lumineuse se trouvant à l'intérieur de la première plage de longueurs d'onde du filtre, ainsi qu'une unité de commande/d'évaluation (14) configurée pour déterminer, à partir d'au moins une image acquise par l'unité de détection optique (11), la présence du gaz ou du mélange gazeux (2), la répartition du gaz ou du mélange gazeux (2) dans le champ de vision de l'unité de détection optique (11), la composition du gaz ou du mélange gazeux (2) et/ou une concentration des composants du mélange gazeux (2), en ce que l'unité de commande/d'évaluation provoque les étapes suivantes :
a. fonctionnement du dispositif (1) dans un mode de détection passif, la première source lumineuse (12) étant éteinte dans le mode de détection passif et l'unité de détection optique (11) acquérant une première image,
b. évaluation de la première image acquise et détermination de la position d'un gaz ou d'un mélange gazeux (2) dans le champ de vision de l'unité de détection optique (11),
c. fonctionnement du dispositif (1) dans un mode de détection actif, la première source lumineuse (12) étant allumée dans le mode de détection actif et émettant de la lumière à une première longueur d'onde actuelle :
i. orientation du dispositif (1) avec l'unité de détection optique (11) vers la position déterminée et acquisition d'une deuxième image au moyen de l'unité de détection optique (11) ;
ii. acquisition d'une troisième image au moyen de l'unité de détection optique (11) à la position déterminée, la première source lumineuse (12) émettant une deuxième longueur d'onde actuelle différente de la première longueur d'onde actuelle ;
d. détermination de la composition du gaz ou du mélange gazeux (2), ou identification du gaz ou du mélange gazeux (2), par évaluation de la deuxième image et de la troisième image, la source lumineuse (12) émettant successivement la première longueur d'onde actuelle et une ou plusieurs autres longueurs d'onde actuelles, l'unité de détection optique (11) relevant une ligne d'absorption du gaz ou du mélange gazeux, sur la base de laquelle les propriétés du gaz ou du mélange gazeux (2) peuvent être déterminées avec précision, au moins une autre image étant acquise en présence de chacune des autres longueurs d'onde actuelles, ladite autre image ou lesdites autres images étant également évaluées pour déterminer la composition du gaz ou du mélange gazeux (2) ou identifier le gaz ou le mélange gazeux (2).

2. Dispositif (1) selon la revendication 1, l'unité de commande/d'évaluation (14) étant configurée pour synchroniser un réglage d'au moins une longueur d'onde actuelle de la première source lumineuse (12) avec un déclenchement de l'unité de détection optique (11) pour l'acquisition d'au moins une image.

3. Dispositif (1) selon la revendication 1, l'unité de commande/d'évaluation (14) étant configurée pour régler successivement la première source lumineuse (12) sur au moins deux longueurs d'onde actuelles différentes l'une de l'autre, la première source lumineuse étant configurée pour déclencher l'unité de détection optique (11) chaque fois que la première source lumineuse (12) est réglée sur les longueurs d'onde actuelles respectives.

4. Dispositif (1) selon au moins l'une des revendications précédentes, le dispositif (1) comprenant un ou plusieurs autres filtres optiques (13'), les autres filtres optiques (13') présentant chacun une autre plage de longueurs d'onde de filtre différente les unes des autres et de la première plage de longueurs d'onde de filtre.

5. Dispositif (1) selon la revendication 4, comprenant en outre une roue à filtres (15), qui comporte le premier filtre optique (13) et le ou les autres filtres optiques (13, 13'), la roue à filtres (15) étant configurée pour disposer sélectivement, par rotation de la roue à filtres (15), le premier filtre optique (13), l'un des autres filtres optiques (13') ou aucun filtre optique dans le champ de vision de l'unité de détection optique (11).

6. Dispositif (1) selon la revendication 4, le dispositif (1) comprenant un logement de filtre (16) destiné à disposer un filtre optique (13, 13') dans le champ de vision de l'unité de détection optique (11), le premier filtre optique (13), l'un des autres filtres optiques (13') ou aucun filtre optique pouvant être disposé sélectivement dans le logement de filtre (16).

7. Dispositif (1) selon au moins l'une des revendications précédentes, la première plage de longueurs d'onde de la première source lumineuse (12) étant à bande étroite.

8. Dispositif (1) selon au moins l'une des revendications précédentes, la première plage de longueurs d'onde se situant dans le domaine infrarouge ou dans le domaine UV.

9. Dispositif (1) selon au moins l'une des revendications précédentes, comprenant en outre une ou plusieurs autres sources lumineuses (12'), les autres sources lumineuses (12') présentant chacune une autre plage de longueurs d'onde différente les unes des autres et de la première plage de longueurs d'onde, l'autre source lumineuse (12'), ou l'une des autres sources lumineuses (12'), pouvant être mise en marche et arrêtée sélectivement et celle-ci, ou l'unité de détection optique (11), étant disposée de telle sorte que sa lumière émise atteigne le champ de vision de l'unité de détection optique (11).

10. Dispositif (1) selon au moins l'une des revendications précédentes, la première source lumineuse (12), ou les autres sources lumineuses (12'), étant un laser accordable, notamment un laser à diode ou un laser à cascade quantique.

11. Dispositif (1) selon au moins l'une des revendications précédentes, comprenant une unité de manipulation optique configurée de telle sorte qu'elle élargisse la lumière émise par la première source lumineuse (12), ou par l'autre source lumineuse (12'), ou par l'une des autres sources lumineuses (12'), de manière qu'elle atteigne l'ensemble du champ de vision de l'unité de détection optique (11), ou configurée de telle sorte que la lumière émise par la première source lumineuse (12) soit ponctuelle et soit déplacée en trame sur le champ de vision de l'unité de détection optique (11), l'unité de commande/d'évaluation (14) étant configurée pour acquérir une pluralité d'images à différentes positions de la lumière dans la trame.

12. Procédé de détection d'un gaz ou d'un mélange gazeux multicomposant (2) au moyen d'un dispositif (1) selon l'une des revendications 1 à 11, comprenant :
- fonctionnement du dispositif (1) dans un mode de détection passif, la première source lumineuse (12) étant éteinte dans le mode de détection passif et l'unité de détection optique (11) acquérant une première image,
- évaluation de la première image acquise et détermination de la position d'un gaz ou d'un mélange gazeux (2) dans le champ de vision de l'unité de détection optique (11),
- fonctionnement du dispositif (1) dans un mode de détection actif, la première source lumineuse (12) étant allumée dans le mode de détection actif et émettant de la lumière à une première longueur d'onde actuelle :
i. orientation du dispositif (1) avec l'unité de détection optique (11) vers la position déterminée et acquisition d'une deuxième image au moyen de l'unité de détection optique (11) ;
ii. acquisition d'une troisième image au moyen de l'unité de détection optique (11) à la position déterminée, la première source lumineuse (12) émettant une deuxième longueur d'onde actuelle différente de la première longueur d'onde actuelle ;
- détermination de la composition du gaz ou du mélange gazeux (2), ou identification du gaz ou du mélange gazeux (2), par évaluation de la deuxième image et de la troisième image, la source lumineuse (12) émettant successivement la première longueur d'onde actuelle et une ou plusieurs autres longueurs d'onde actuelles, l'unité de détection optique (11) relevant une ligne d'absorption du gaz ou du mélange gazeux, sur la base de laquelle les propriétés du gaz ou du mélange gazeux (2) peuvent être déterminées avec précision, au moins une autre image étant acquise en présence de chacune des autres longueurs d'onde actuelles, ladite autre image ou lesdites autres images étant également évaluées pour déterminer la composition du gaz ou du mélange gazeux (2) ou identifier le gaz ou le mélange gazeux (2).

13. Procédé selon la revendication 12, dans lequel, ensuite, la ou les autres sources lumineuses (12') sont successivement allumées, plusieurs longueurs d'onde actuelles étant notamment réglées successivement, et l'unité de détection optique (11) acquérant une ou plusieurs autres images actuelles, ladite autre image ou lesdites autres images étant également évaluées pour déterminer la composition du gaz ou du mélange gazeux (2) ou identifier le gaz ou le mélange gazeux (2).

14. Procédé selon au moins l'une des revendications 12 ou 13, dans lequel, pour chacune des autres sources lumineuses (12'), l'un des autres filtres optiques est activé.
